# EUROPEAN PATENT SPECIFICATION

(11) **EP 1 594 978 B1**
(45) Date of publication and mention of the grant of the patent: **25.07.2007**
(21) Application number: 04712486.2
(22) Date of filing: 19.02.2004
(51) Int. Cl.: C12Q 1/18, A61P 31/04

(54) **ASSAY FOR THE IDENTIFICATION OF PROTEIN SIC INHIBITORS**
VERFAHREN ZUR IDENTIFIZIERUNG VON PROTEIN SIC INHIBITOREN
ANALYSE POUR L'IDENTIFICATION D'UN INHIBITEUR DE LA PROTEINE SIC

(30) Priority: 19.02.2003 GB 0303809
(43) Date of publication of application: 16.11.2005
(73) Proprietor: Hansa Medical AB, 205 02 Malmö (SE)
(72) Inventor: AAKESSON, Per, SE-21618 Limhamm (SE); BJÖRCK, Lars, 223 50 Lund (SE); SJÖHOLM, Anders, 222 20 Lund (SE)
(74) Representative: Roques, Sarah Elizabeth
(86) International application number: PCT/EP2004/001581
(87) International publication number: WO 2004/074807

(56) References cited:
- WO-A-02/072624
- HOE NANCY P ET AL: "Insight into the molecular basis of pathogen abundance: Group A Streptococcus inhibitor of complement inhibits bacterial adherence and internalization into human cells" 28 May 2002 (2002-05-28), PROCEEDINGS OF THE NATIONAL ACADEMY OF SCIENCES OF THE UNITED STATES OF AMERICA, VOL. 99, NR. 11, PAGE(S) 7646-7651 , XP002289162 ISSN: 0027-8424 page 7647, column 1, paragraph 1 page 7647, column 1, paragraph 4 page 7648, column 2, paragraph 2 page 7649, column 1, paragraph 1
- FERNIE-KING B A ET AL: "STREPTOCOCCAL INHIBITOR OF COMPLEMENT INHIBITS TWO ADDITIONAL COMPONENTS OF THE MUCOSAL INNATE IMMUNE SYSTEM: SECRETORY LEUKOCYTE PROTEINASE INHIBITOR AND LYSOZYME" INFECTION AND IMMUNITY, AMERICAN SOCIETY OF MICROBIOLOGY, WASHINGTON, DC, US, vol. 70, no. 9, September 2002 (2002-09), pages 4908-4916, XP001097330 ISSN: 0019-9567
- FERNIE-KING B ET AL: "Subversion of the innate immune response by micro-organisms" November 2002 (2002-11), ANNALS OF THE RHEUMATIC DISEASES, VOL. 61, NR. SUPPLEMENT 2, PAGE(S) II8-II12 , XP002289161 ISSN: 0003-4967 page II11, column 1 - column 2
- AKESSON P ET AL: "Protein SIC, a novel extracellular protein of Streptococcus pyogenes interfering with complement function" JOURNAL OF BIOLOGICAL CHEMISTRY, AMERICAN SOCIETY OF BIOLOGICAL CHEMISTS, BALTIMORE, MD, US, vol. 271, no. 2, 1996, pages 1081-1088, XP002218452 ISSN: 0021-9258
- LUKOMSKI SLAWOMIR ET AL: "Nonpolar inactivation of the hypervariable streptococcal inhibitor of complement gene (sic) in serotype M1 Streptococcus pyogenes significantly decreases mouse mucosal colonization" INFECTION AND IMMUNITY, vol. 68, no. 2, February 2000 (2000-02), pages 535-542, XP002289164 ISSN: 0019-9567
- SCHMIDTCHEN ARTUR ET AL: "Dermatan sulphate is released by proteinases of common pathogenic bacteria and inactivates antibacterial alpha-defensin" MOLECULAR MICROBIOLOGY, BLACKWELL SCIENTIFIC, OXFORD, GB, vol. 39, no. 3, February 2001 (2001-02), pages 708-713, XP002194955 ISSN: 0950-382X
- SCHMIDTCHEN ARTUR ET AL: "Proteinases of common pathogenic bacteria degrade and inactivate the antibacterial peptide LL-37" October 2002 (2002-10), MOLECULAR MICROBIOLOGY, VOL. 46, NR. 1, PAGE(S) 157-168 , XP002289163 ISSN: 0950-382X the whole document

## Description

### Field of the Invention

The invention relates to assays for antibacterial agents, and in particular antibacterial agents useful in the treatment of streptococcal infections.

### Background to the Invention

*S. pyogenes* is one of the most common and important human bacterial pathogens. It causes relatively mild infections such as pharyngitis (strep throat) and impetigo, but also serious clinical conditions like rheumatic fever, post-streptococcal glomerulonephritis, necrotizing fasciitis, septicemia, and a toxic-shock syndrome. Increases in the number of life-threatening systemic S. *pyogenes* infections have been reported world-wide since the late 1980's, and have attracted considerable attention and concern. Based on the highly polymorphic M protein, a surface protein of *S. pyogenes,* isolates are divided into more than 100 serological subtypes and systemic infections are most frequently caused by organisms of the M1 serotype.

Protein SIC was originally isolated from the growth medium of an M1 strain (1). All strains of the M1 serotype secrete SIC and so do M57 organisms, whereas strains of 53 other serotypes were found to lack the *sic* gene (1). Subsequent work has identified *sic* homologues also in M12 and M55 strains (1). The name SIC stands for streptococcal inhibitor of complement, as the protein incorporates into the membrane attack complex (MAC) of complement and inhibits complement-mediated lysis of sensitized erythrocytes (1). This inhibition of MAC was recently shown to be the result of SIC preventing uptake of C567 onto cell membranes (3). A remarkable property of SIC was reported by Stockbauer *et al.* (4). They found that the sequences of a large number of *sic* genes from different strains of the M1 serotype, showed a unique degree of variation, which is in striking contrast to the lack of M1 protein variation. Moreover, in a mouse model of infection, Hoe *et al.* (5) discovered that SIC variants arise rapidly on mucosal surfaces by natural selection. They also reported that the inhibition of complement-mediated lysis by SIC, was not affected in the new SIC variants arising from natural selection, suggesting that complement inhibition is not the only function of SIC.

Complement belongs to the innate immune system and antibacterial peptides represent another important part of this defence system. These peptides, originally described in silk worms, play important roles in the clearance of bacteria at biological boundaries susceptible for infection (6-9).

### Summary of the Invention

In accordance with the present invention, there is provided an assay method for an anti-bacterial agent comprising:
(a) providing as a first component protein SIC;
(b) providing as a second component an antibacterial peptide selected from α-defensin, LL-37 or a mixture thereof;
(c) contacting the first component with a test substance in the presence of the second component; and
(d) determining the interaction or activity of the first component with the second component to determine thereby whether a test substance is an effective anti-bacterial agent.

Inhibitors of protein SIC, for example identified in accordance with the present invention may be used in the treatment of prophylaxis of *S*. *pyogenes* infection. The protein SIC inhibitors may be used together with antibacterial peptides to treat *S. pyogenes* infection.

### Description of the Figures

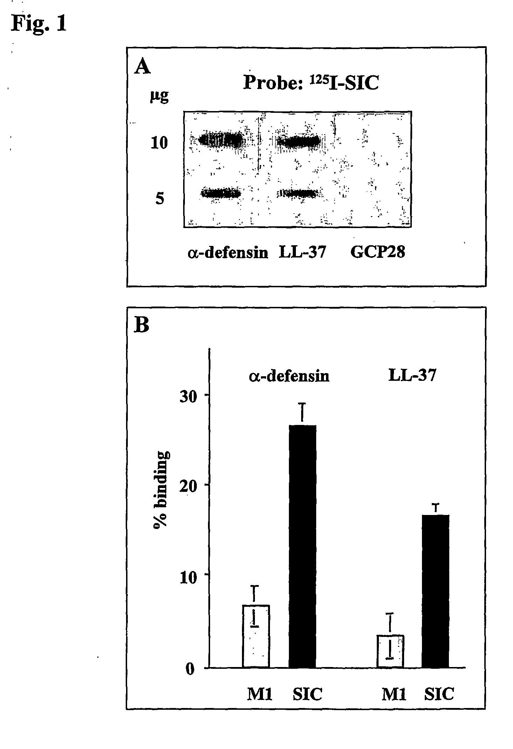

FIG. 1. **Sic interacts with antibacterial peptides.** (A) Various amounts of the antibacterial peptides α-defensin and LL-37, and the peptide GCP derived from human H-kininogen, were applied to a PVDF membrane. The membrane was incubated with radiolabelled protein SIC (2 x 10⁵ cpm/ml) for 3 h and autoradiographed for 3 days. (B) Microtiter plates were coated with protein SIC or M1 protein at 2.9 nM, followed by incubation with α-defensin or LL-37 (58 nM). Binding was detected with specific antibodies against α-defensin and LL-37, respectively. The bars represent the mean ± SEM of at least three experiments.

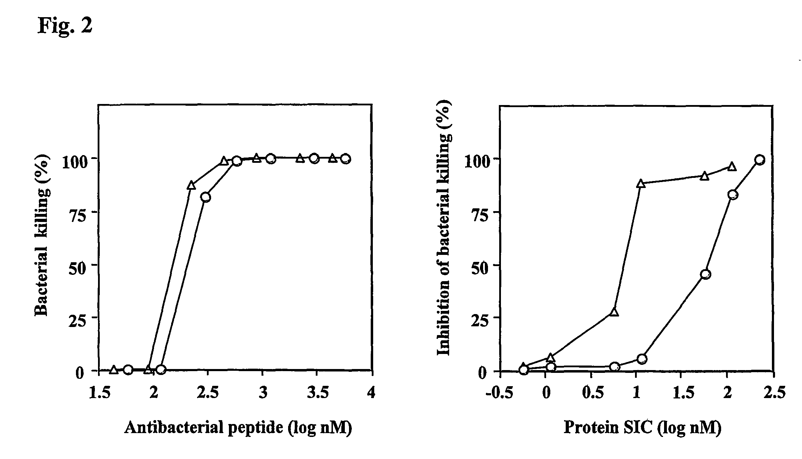

**FIG. 2. SIC protects *S. pyogenes* against antibacterial peptides.** AP1 bacteria (2 x 10⁶ cfu/ml) were incubated with the antibacterial peptides α-defensin (O) or LL-37 (Δ) at indicated concentrations for 2 h at 37°C and cfus were determined (left panel). The bactericidal effect of α-defensin (O) or LL-37 (Δ), at a concentration of 448 nM, was inhibited with various concentrations of protein SIC (right panel). Experiments were repeated at least three times and representative experiments are shown.

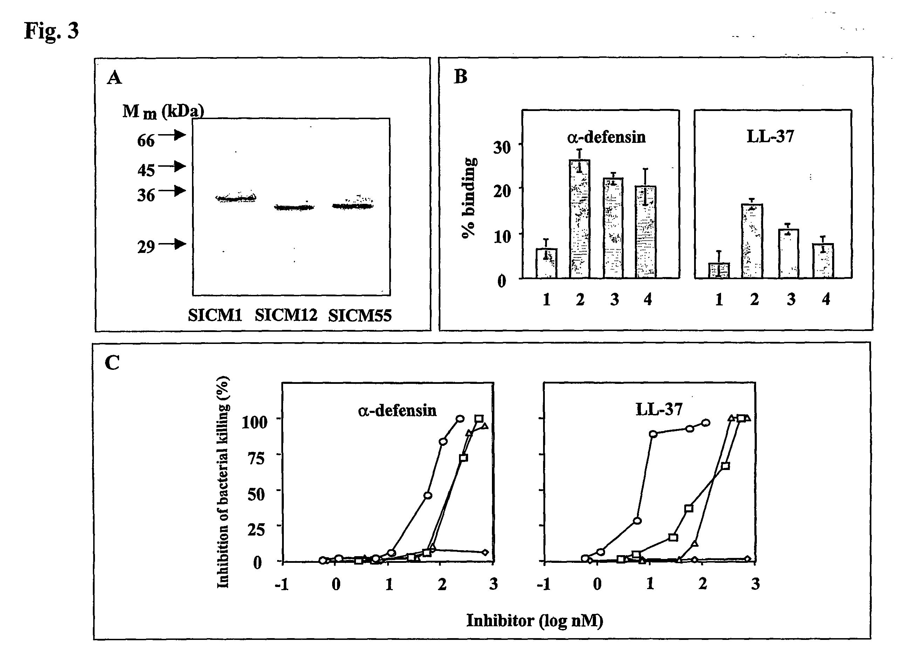

FIG. 3. **Different SIC homologues block the bactericidal effect of α-defensin and LL-37.** (A) Different homologues of protein SIC (1 µg) were subjected to SDS-PAGE (10% gel) and stained with Coomassie blue. Protein SICM1 is purified from *S. pyogenes* strain AP1; protein SICM12 from S. *pyogenes* strain AP12; and protein SICM55 from *S*. *pyogenes* strain W38. (B) Microtiter plates were coated with the different homologues of protein SIC shown in panel A, or with M1 protein, at 2.9 nM, followed by incubation with α-defensin or LL-37 (58 nM). Binding was detected with specific antibodies against α-defensin and LL-37, respectively. Lane 1: M1 protein; Lane 2: protein SICM1; Lane 3: protein SICM12; Lane 4: protein SICM55. The bars represent the mean ± SEM of at least three experiments. (C) AP1 bacteria were incubated with antibacterial peptides (448 nM) for 2h (α-defensin) or 1 h (LL-37) in the presence of various amounts of proteins SICM1 (O), SICM12 (□), SICM55 (Δ), or M1 protein (◊). The different preparations of protein SIC shown in panel A were used as inhibitors. Experiments were repeated at least three times and representative experiments are shown.

### Detailed Description of the Invention

We have shown that protein SIC plays a role in inactivating anti-microbial peptides. The ability to inactivate these anti-microbial peptides increases the virulence of bacterial infection. This activity presents a novel target for the identification of antibacterial agents, in particular which can be used to inhibit the activity of protein SIC and therefore allow endogenous or administered antibacterial peptides to maintain their activity.

The invention provides methods for identifying an anti-bacterial agent. A suitable method of the invention comprises (a) providing as a first component, protein SIC; (b) providing as a second component an antibacterial peptide; (c) contacting the two components with a test substance; and (d) determining whether the test substance is capable of modulating the interaction between protein SIC and the antibacterial peptide.

Protein SIC or a functional variant thereof is provided as one component for use in the assays of the invention. Protein SIC can be provided from any suitable source. The amino acid sequence of protein SIC from the M1 strain of *S*. *pyogenes* is set out in SEQ ID NO: 1. Any suitable protein SIC can be provided including protein SIC derived from M1 serotype, M57 serotype, M12 serotype and M55 serotype or any other serotypes of *S. pyogenes* which expresses protein SIC. Examples of SIC genes and the encoded proteins are described in Stockbauer et al (4) and are suitable proteins for use in the methods of the present invention.

A functional variant of protein SIC has a sequence similar to that of SEQ ID NO: 1 and retains the ability to interact with and/or to interfere with the activity of antibacterial peptides. Typically, the activity of a functional variant of SIC is substantially the same as that wild type protein SIC. Alternatively, the activity may be greater or less than that of protein SIC. For example, a functional variant may have at least 90% activity, at least 80% activity or at least 70% activity of protein SIC of SEQ ID NO: 1 with respect to its ability to interact with and/or inactivate antibacterial peptides.

A.functional variant typically comprises a sequence similar to that set out in the amino acid sequence of SEQ ID NO: 1.

Thus a functional variant will generally have at least 60%, at least 70%, at least 80%, at least 90%, at least 95%, at least 98 or at least 99% sequence identity to the protein SIC having the sequence set out as the amino acid sequence of SEQ ID NO: 1, calculated over the full length of those sequences. In the alternative, a functional variant will generally have at least 60%, at least 70%, at least 80%, at least 90%, at least 95%, at least 98% or at least 99% sequence identity to protein SIC derived from another strain of *S*. *pyogenes.* The UWGCG Package provides the BESTFIT program which can be used to calculate identity (for example used on its default settings) (Devereux et al (1984) Nucleic Acids Research 12, p387-395). The PILEUP and BLAST algorithms can be used to calculate identity or line up sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Centre for Biotechnology Information (http://www.ncbi.nlm.nih.gov/).

A functional variant may be a naturally occurring sequence, such as a related gene from another strain of the M1 serotype, or a variant from another strain of a different serotype such as a strain of the M12, M55 or M57 serotype.

Alternatively, a functional variant may be a non-naturally occurring sequence. A non-naturally occurring functional variant may be a modified version of protein SIC having a modified sequence to that set out as the amino acid sequence of SEQ ID NO: 1, obtained by, for example, amino acid substitution, deletion or addition. Up to 1, up to 5, up to 10, up to 50 or up to 100 amino acid substitutions or deletions, for example, may be made. Thus, a functional variant of the protein SIC may be a fragment of that sequence. Typically, if substitutions are made, the substitutions will be conservative substitutions, for example according to the following Table. Amino acids in the same block in the second column and preferably in the same line in the third column may be substituted for each other. Deletions are preferably deletions of amino acids from one or both ends of the sequence of protein SIC or of deletions of one or more of the repeat regions of protein SIC. Alternatively, deletions are of regions not involved in the interaction or inactivation of antibacterial peptides:

| | | |
|---|---|---|
| ALIPHATIC | Non-polar | GAP |
| | | ILV |
| | Polar-uncharged | C S T M |
| | | NQ |
| | Polar-charged | D E |
| | | KR |
| AROMATIC | | HFWY |

Typically, protein SIC is provided having been produced in recombinant form from a suitable host cell. Alternatively, protein SIC can be provided by providing bacterial cells that express protein SIC endogenously or through the use of recombinant techniques. For example, the assays of the invention may use a suitable strain of *S. pyogenes* or other bacteria which expresses protein SIC. Such bacteria are incubated with an antibacterial peptide in the presence of the test substance. Alternatively, a separate source of protein SIC can be provided which has either been produced by recombinant or other suitable techniques or alternatively has been isolated from protein SIC expressing bacterial strains.

Protein SIC or a functional variant thereof may be fused to a carrier polypeptide. Thus, additional amino acid residues may be provided at, for example, one or both termini of protein SIC or a functional variant thereof for the purpose of providing a carrier polypeptide, by which the polypeptide can be, for example, affixed to a label, solid matrix or carrier. Thus the first component for use in a method of the invention may be in the form of a fusion polypeptide which comprises heterologous sequences. Typically, fusion polypeptides will comprise a polypeptide sequence as described above and a carrier or linker sequence.

Polypeptides may be modified by, for example, addition of histidine residues, a T7 tag or glutathione S-transferase, to assist in their isolation. Alternatively, the carrier polypeptide may, for example, promote secretion of the polypeptide from a cell or target expression of the polypeptide to the cell membrane. Amino acids carriers can be from 1 to 400 amino acids in length or more typically from 5 to 200 residues in length. The polypeptide may be linked to a carrier polypeptide directly or via an intervening linker sequence. Typical amino acid residues used for linking are tyrosine, cysteine, lysine, glutamic acid or aspartic acid.

Suitable polypeptides for use as a first component may be chemically modified, for example, post translationally modified. For example they may be glycosylated or comprise modified amino acid residues. Polypeptides can be in a variety of forms of polypeptide derivatives, including amides and conjugates with polypeptides.

Chemically modified polypeptides also include those having one or more residues chemically derivatized by reaction of a functional side group. Such derivatized side groups include those which have been derivatized to form amine hydrochlorides, p-toluene sulfonyl groups, carbobenzoxy groups, t-butyloxycarbonyl groups, chloroacetyl groups and formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine.

Also included as chemically modified polypeptides are those polypeptides which contain one or more naturally occurring amino acids derivatives of the twenty standard amino acids. For example, 4-hydroxyproline may be substituted for proline or homoserine may be substituted for serine.

Protein SIC or a functional variant thereof and/or other polypeptides used as part of a first component may carry a revealing label. Suitable labels include radioisotopes such as ³²P or ³⁵S, fluorescent labels, enzyme labels, or other protein labels such as biotin.

Protein SIC or a functional variant thereof and/or other polypeptides used as part of a first component may be expressed using recombinant DNA techniques. For example, suitable polypeptides may be expressed in, for example, bacterial or insect cell lines (see, for example, Munger et al., 1998, Molecular Biology of the Cell, 9, 2627-2638). Such polypeptides are produced by providing a polynucleotide encoding protein SIC, such as a polynucleotide of SEQ ID NO: 2. Such polynucleotides are provided with suitable control elements, such as promoter sequences, and provided in expression vectors and the like for expression of protein SIC. Also, suitable polypeptides may be isolated biochemically from any suitable bacteria.

Alternatively, polypeptides may be chemically synthesized. Synthetic techniques, such as a solid-phase Merrifield-type synthesis, may be preferred for reasons of purity, antigenic specificity, freedom from unwanted side products and ease of production. Suitable techniques for solid-phase peptide synthesis are well known to those skilled in the art (see for example, Merrifield et al., 1969, Adv. Enzymol 32, 221-96 and Fields et al., 1990, Int. J. Peptide Protein Res, 35, 161-214). In general, solid-phase synthesis methods comprise the sequential addition of one or more amino acid residues or suitably protected amino acid residues to a growing peptide chain.

The second component comprises the antibacterial peptides human neutrophil α-defensin and LL37. The sequences of α-defensin and or LL-37 are set out in SEQ ID NO: 3 and SEQ ID NO: 4. These proteins may be produced by any suitable technique including recombinant techniques and chemical synthesis as described in more detail for the first component polypeptide.

The assay can be carried out according to any suitable protocol. Preferably, the assay is adapted so that it can be carried out in a single reaction vessel more preferably can be carried out in a single well of a plastics Microtiter plate and thus can be adapted for high throughput screening.

The assays of the present invention monitor the interaction or activity between protein SIC and an antibacterial peptide. The interaction can be detected in a simple binding assay, for example by coating one of the components to a solid support and monitoring for binding of the second component, for example, using a suitable label such as a radioactive label or fluorescent label on the component. Alternative assays of the invention monitor for the activity of protein SIC on the antibacterial peptides. For example, the two components may be incubated together in the presence of a test substance and the extent, if any, of the reduction in activity of the antibacterial peptide can be monitored.

In an example of a suitable assay format, the antibacterial activity of the antibacterial peptides can be monitored. This activity can be monitored by any suitable means. The activity of the antibacterial peptide can be determined after the peptide has been incubated with a test substance and protein SIC, for example, by monitoring for the antibacterial effect of the antibacterial peptide in the presence of bacteria. Assays can be performed in which bacteria are also present in the assay sample during the course of the assay and the growth of the bacteria can be monitored during the course of the assay. Alternatively, after incubation of protein SIC and antibacterial peptide in the presence of the test substance, the assay sample can be contacted with bacteria in a separate vessel. The assays of activity or degradation of antibacterial peptides can be carried out at selected time points following addition of the test substance to determine the cause of the reaction. The components may be added together in any order, although typically protein SIC and the antibacterial peptide are only incubated together in the presence of the test substance.

In one embodiment, protein SIC is provided by *S. pyogenes* or other recombinant bacteria which express protein SIC and the activity of the antibacterial peptides is monitored by monitoring the growth of those bacteria, in the presence of a test substance. Assays may be carried out in which additional amounts of an antibacterial peptide are added to the sample to establish the amount of antibacterial peptide that is required to be added to obtain an antibacterial effect, to thereby determine the activity of initial quantity of antibacterial peptides. Such an assay can thus establish the effect of the test substance on the activity of protein SIC on the antibacterial peptide.

Suitable control experiments may be carried out. For example, assays may be carried out in the absence of a test substance to monitor for the activity of protein SIC on the antibacterial peptides in the absence of a test substance. Assays may also be carried out in the absence of antibacterial peptides, to distinguish between agents which inhibit bacterial growth generally, rather than having a specific activity on the protein SIC activity on antibacterial peptides.

Suitable test products which can be tested in the above assays include combinatorial libraries, defined chemical entities, peptide and peptide mimetics, oligonucleotides and natural product libraries, such as display (e.g. phage display libraries) and antibody products. Typically, organic molecules will be screened, preferably small organic molecules which have a molecular weight of from 50 to 2500 daltons. Candidate products can be biomolecules including, saccharides, fatty acids, steroids, purines, pyrimidines, derivatives, structural analogs or combinations thereof. Candidate agents are obtained from a wide variety of sources including libraries of synthetic or natural compounds. Known pharmacological agents may be subjected to directed or random chemical modifications, such as acylation, alkylation, esterification, amidification, etc. to produce structural analogs.

Test substances may be used in an initial screen of, for example, 10 substances per reaction, and the substances of these batches which show inhibition or stimulation tested individually. Test substances may be used at a concentration of from 1nM to 1000µM, preferably from 1µM to 100µM, more preferably from 1µM to 10µM.

An inhibitor of protein SIC activity/interaction with antibacterial peptide is one which produces a measurable reduction on the activity or interaction in the assay as described above. An inhibitor of the interaction between protein SIC and an antibacterial peptide is one which causes the degree of interaction and in particular the activity of protein SIC on antibacterial peptide to be reduced or substantially eliminated, as compared to the degree of interaction between the two, in the absence of that inhibitor. Preferred inhibitors are those which inhibit the interaction/activity between protein SIC and the antibacterial peptide by at least 10%, at least 20%, at least 30%, at least 40%, at least 50%, at least 60%, at least 70%, at least 80%, at least 90%, at least 95% or at least 99% at a concentration of the inhibitor of 1 µgml⁻¹, 10 µgml⁻¹, 100 µgml⁻¹, 500 µgml⁻¹, 1 mgml⁻¹, 10 mgml⁻¹, 100mg ml⁻¹. The percentage inhibition represents the percentage decrease in the interaction between protein SIC and the antibacterial peptide in a comparison of assays in the presence and absence of the test substance. Any combination of the above mentioned degrees of percentage inhibition and concentration of inhibitor may be used to define an inhibitor of the invention, with greater inhibition at lower concentrations being preferred.

Modulators of the invention may be in substantially purified form. They may be in substantially isolated form, in which case they will generally comprise at least 80% e.g. at least 90, 95, 97 or 99% by weight of the dry mass in the preparation. The product is typically substantially free of other cellular components. The product may be used in such a substantially isolated, purified or free form in the method or be present in such forms in a kit.

The formulation of a modulator for use in preventing or treating bacterial infection will depend upon factors such as the nature of the exact modulator, whether a pharmaceutical or veterinary use is intended, etc. A modulator may be formulated for simultaneous, separate or sequential use.

A modulator is typically formulated for administration in the present invention with a pharmaceutically acceptable carrier or diluent. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate; glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

### Examples

*Bacterial strains, and purification of protein SIC-* The *S. pyogenes* strains AP1 (40/58) of serotype M1 and AP12 (1/71) of serotype M12 were from the WHO Collaborating Centre for Reference and Research on Streptococci, Prague, Czech Republic. The S. *pyogenes* strain W38 (GT 71-154) of serotype M55 was from the late Dr. L.W. Wannamaker. Bacteria were grown in Todd-Hewitt broth (TH; Difco, Detroit, MI) at 37°C. Protein SIC was purified from the S. *pyogenes* strains AP1, AP12 and W38 as described (1) by precipitation of the culture medium with 30% ammonium sulfate, followed by ion-exchange chromatography on Mono Q (Pharmacia-Biotech, Uppsala, Sweden). Fractions containing protein SIC were combined, dialysed against 2 mM NH₄HCO₃ and freeze-dried. For the antimicrobial assay (see below) protein SIC was dissolved in 10 mM Tris-HCl, pH 7.5, containing 5 mM glucose.

*Proteins, peptides, antibodies and radiolabelling-* Recombinant M1 protein was prepared as described previously (10), α-defensin (HNP-1), ACYCRIPACIAGERRYGTCIYQGRLWAFCC (Mw 3442) was purchased from Sigma, and LL-37, LLGDFFRKSKEKIGKEFKRIVQRIKDFLRNLVPRTES (Mw 4492) was synthesized by Innovagen AB, Lund, Sweden. The peptide GCP28, GCPRDIPTNSPELEETLTHTITKLNAEN, based on a sequence in human kininogen has been described previously (11) and was a kind gift from Dr. Heiko Herwald, Lund University, Lund, Sweden. Mouse monoclonal α-defensin antibodies were from Bachem AG, and rabbit polyclonal LL-37 antibodies were from Innovagen AB. Antisera against protein SIC was raised in rabbits. Protein SIC was labelled with ¹²⁵I using the chloramine T method (12).

*Antimicrobial assay-* AP1 bacteria were grown to mid-log phase in TH broth, washed and diluted in 10 mM Tris-HCl, pH 7.5, containing 5 mM glucose. 50 µl bacteria (2 x 10⁶ colony forming units (cfu)/ml) were incubated together with α-defensin or LL-37 at various concentrations for 2 h at 37°C. In subsequent experiments, bacteria were incubated with α-defensin and LL-37 at a concentration of 448 nM together with different concentrations of protein SIC or M1 protein and the reactions were carried out for 2 h (α-defensin) or 1 h (LL-37). To quantitate the bactericidal activity serial dilutions of the incubation mixtures were plated on TH agar, incubated overnight at 37°C, and the number of cfus were determined.

*Bacterial growth assay-* AP1 bacteria were grown to stationary phase in TH broth. 200 µl of TH was inoculated with 5 µl of the bacterial suspension in 96-well plates (Falcon) at 37°C. At early logarithmic phase various amounts of LL-37 was added and growth was followed by measuring the absorbance at 490 nm (using a BioRad 550 microplate reader). The amount of protein SIC in the growth medium was estimated by ELISA (see below).

*Slot-binding and SDS-polyacrylamide gel electrophoresis-* Peptides were applied to polyvinylidene difluoride (PVDF) membranes (Immobilon, Millipore) using a Milliblot-D system (Millipore). Membranes were blocked in TBS (0.05 M Tris-HCl, pH 7.5, 0.15 M NaCl) containing 3% bovine serum albumin (BSA), incubated with ¹²⁵I-labelled protein SIC for 3 h, and washed with TBS containing 0.05% Tween-20. Autoradiography was carried out using Kodak x-Omat AR films and regular intesifying screens. SDS-polyacrylamide gel electrophoresis (PAGE) was performed as described by Laemmli (13) using a polyacrylamide concentration of 10% and 3.3% cross-linking. Samples were boiled for 3 min in sample buffer containing 2% SDS and 5% 2-mercaptoethanol. Gels were stained with Coomassie blue.

*ELISA*-Indirect ELISA was performed by coating microtiter plates (Maxisorb, NUNC, Denmark) over night with proteins in serial dilutions (starting at 58 nM). The plates were washed in PBS containing 0.05% Tween-20 (PBST), blocked in PBST containing 2% BSA for 30 min and incubated with 580 nM α-defensin or LL-37 for 1 h. Bound antibacterial peptides were detected with specific antbodies against α-defensin (1: 2000) or LL-37 (1:5000), and binding was visualized by a horseradish peroxidase-conjugated secondary antibody against mouse or rabbit IgG (1:3000). All incubations were performed at 37°C for 1 h followed by a washing step. Substrate solution, 0.1 % (w/v) diammonium-2,2-azino-bis-(3-ethyl-2,3-dihydrobenzthiazoline)-6-sulfonate (ABTS), 0.012% (v/v) H₂O₂ in 100mM NaH₂PO₄, pH4.5, was added and the change in absorbance at 405 nm was determined after 5 min. To determine the concentration of SIC in growth medium plates coated with AP1 growth medium were incubated with antibodies against protein SIC (1:1000) followed by a secondary antibody against rabbit IgG (1:3000). Visualization of binding was detected as above and absorbance was determined after 30 min.

### Results

Two major and well-characterized human antibacterial peptides, α-defensin (HNP-1) and LL-37, were used in this investigation. These peptides have broad antibacterial activity against both Gram-positive and Gram-negative bacteria. α-defensin (HNP-1) is found in the azurophilic granules of human neutrophils. Analogues to neutrophil α-defensins are produced also by intestinal Paneth cells (14,15). LL-37 is produced by neutrophils and epithelial cells. α-defensin and LL-37 are both found in extracellular fluids, including wound fluid, and the two peptides act synergistically on target bacteria (16).

In *S. pyogenes,* the *sic* gene is part of the so-called *mga* regulon. Like the other genes of the *mga* regulon, *sic* is expressed at an early growth phase (1), suggesting that SIC will be secreted as soon as *S. pyogenes* bacteria carrying the *sic* gene starts to grow on an epithelial surface. To investigate whether α-defensin and LL-37 have affinity for SIC, these peptides and a control peptide (GCP28) based on a sequence in H-kininogen (11), were applied to Immobilon filters which were probed with ¹²⁵I-labelled SIC (if not indicated otherwise SIC is from the M1 strain AP1). Figure 1A shows that SIC interacts with the antibacterial peptides, an observation which was confirmed also by experiments where SIC and M1 protein were applied to plastic wells, followed by the addition of α-defensin or LL-37 and antibodies to the peptides. M1 protein was chosen as a control. It was isolated from the same strain of *S. pyogenes* (AP1) as SIC (1), and although the protein is predominantly associated with the cell wall, it is also released from the bacterial surface by proteolytic cleavage (17). In the experiments summarized in Figure 1B, α-defensin and LL-37 showed affinity for SIC, whereas the interaction with M1 protein was at background level.

Next we investigated the antibacterial effect of α-defensin and LL-37 on the AP1 strain. In these experiments the bacteria were washed and resuspended in buffer prior to the addition of the peptides to exclude that SIC was present during the incubation period. As shown in the left panel of Figure 2, the peptides killed the bacteria. The concentration required for 100% killing was approximately 0.4 µM for both peptides. The inhibitory effect of SIC was then tested at a bactericidal concentration (0.448 µM) of the peptides and the results (see right panel, Fig. 2) show that SIC blocks the antibacterial activity of α-defensin and LL-37. The inhibition curves indicate that SIC is about ten times more efficient in blocking LL-37 than α-defensin. M1 protein was also tested (at a maximum of 0.72 µM) but showed no inhibitory activity.

When grown to stationary phase, the growth medium of *S. pyogenes* of the M1 serotype contains large quantities (10-15 µg/ml) of protein SIC (1) and in a series of experiments we investigated whether SIC produced by growing M1 bacteria, could protect the organisms against LL-37 (these experiments required substantial amounts of antibacterial peptide which is why α-defensin was not tested). The AP1 strain was grown to early logarithmic growth phase where the concentration of SIC was 4-5µg/ml growth medium as determined by ELISA. At this point different amounts of LL-37 were added. Compared to the experiments where the bacteria were washed to remove SIC before the addition of LL-37 (see Fig. 2), the concentration of LL-37 required to kill 50% of the bacteria, was more than 50 times higher (11.1 µM compared to 0.2µM). The results suggest that SIC secretion provides effective protection against antibacterial peptides already at an initial stage of infection.

As mentioned, SIC homologues are produced by *S. pyogenes* strains of M serotypes 1, 12, 55, and 57 (1, 2). Using the same isolation protocol as for SIC from M1 bacteria (1), SIC was purified from the growth medium of M12 and M55 organisms. Analogous to the M1 strain (1), the M12 and M55 strains produced 10-15 µg SIC/ml growth medium when grown to stationary phase. The purified homologues (Fig. 3A) and M1 protein were then added to microtiter plates and the binding of α-defensin and LL-37 was determined as described above (see Fig. 1B). α-defensin interacted with the three SIC proteins, whereas in this experimental system only SICM1 showed binding of LL-37 clearly above M1 protein, the negative control (Fig. 3B). When tested for their ability to interfere with the antibacterial activity of α-defensin and LL-37, all SIC homologues, but not M1 protein, blocked this activity (Fig. 3C).The inhibition curves show that SICM1 is 5-10 times more potent in this respect than the M12 and M55 homologues. It is noteworthy that the four SIC-producing M serotypes (1, 12, 55, and 57) are all known to be associated with poststreptococcal glomerulonephritis, suggesting a role for SIC in this condition (1, 2). Moreover, the fact that M1 strains dominate in cases of invasive disease and that SICM1 is the most potent inhibitor of α-defensin and LL-37, support the notion that SIC interference with antibacterial peptides facilitates S. *pyogenes* invasion through mucosal and skin barriers.

Research in many laboratories has over the past 10-20 years firmly established the fundamental role played by antibacterial peptides in the initial clearance of pathogenic bacteria. The demonstration that SIC not only interferes with complement function, but also inactivates antibacterial peptides, further underlines the significance of the innate immune system. The data of the present study also emphasize the highly complex molecular interplay between *S. pyogenes* and its human host. Although pathogenicity and virulence are polygenic properties, the results indicate that SIC could represent an important virulence determinant. A previous investigation (18) showed that an isogenic M1 mutant strain in which the *sic* gene had been inactivated, was significantly less efficient in colonizing the throat of mice as compared to the wild-type strain. These data demonstrate that SIC promotes the early stages of infection and this study suggests that inactivation of antibacterial peptides could be the molecular mechanism behind this effect. The results of the present work may also explain the unique variability of the *sic* gene and the fact that the M1 serotype is the serotype most frequently connected with invasive *S. pyogenes* infection.

### References

1. Åkesson, P., Sjöholm, A. G., and Björck, L. (1996) J Biol,. Chem. 271, 1081-1088.
2. Hartas, J., and Sriprakash, K. S. (1999) Microb Pathog 26, 25-33.
3. Fernie-King, B. A:, Seilly, D. J., Willers, C., Wurzner, R., Davies, A., and Lachmann, P. J. (2001) Immunology 103, 390-8.
4. Stockbauer, K. E., Grigsby, D., Pan, X., Fu, Y. X., Mejia, L. M., Cravioto, A., and Musser, J. M. (1998) Proc Natl Acad Sci USA 95,3128-33.
5. Hoe, N. P., Nakashima, K., Lukomski, S., Grigsby, D., Liu, M., Kordari, P., Dou, S. J., Pan, X., Vuopio-Varkila, J., Salmelinna, S., McGeer, A., Low, D. E., Schwartz, B., Schuchat, A., Naidich, S., De Lorenzo, D., Fu, Y. X., and Musser, J. M. (1999) Nat med 5, 924-9.
6. Boman, H.G. (2000) Immunol. Rev. 173, 5-16.
7. Lehrer, R. I., and Ganz, T. (1999) Curr. Opin. Immunol. 11, 23-27.
8. Schröder, J.-M., and Harder, J. (1999) Int J Biochem Cell Biol 31, 645-651.
9. Selsted, M. E., and Ouellette, A. J. (1995) Trends cell Biol 5, 114-119.
10. Åkesson, P., Schmidt, K.-H., Cooney, J., and Björck, L. (1994) Biochem. J. 300, 877-886.
11. Herwald, H., Hasan, A. A. K., J., G.-Z., Schmaier, A. H., and Müller-Ester1, W. (1995) J. Biol. Chem. 270, 14634-14642.
12. Greenwood, F. C., Hunter, W. M., and Glover, J. S. (1963) Biochem. J. 89, 114-123.
13. Laemmli, U. K. (1970) Nature 227, 680-685.
14. Ouellette, A. J., and Selsted, M. E. (1996) FASEB J 10, 1280-1289.
15. Ayabe, T., Satchell, D. P., Pesendorfer, P., Tanabe, H., Wilson, C. L., Hagen, S. J., and Ouellette, A. J. (2002) J. Biol Chem 227, 5219-28.
16. Nagaoka, I., Hirota, S., Yomogida, S., Ohwada, A., and Hirata, M. (2000) Inflamm Res 49, 73-9.
17. Berge, A., and Björck, L. (1995) J. Biol. Chem. 270, 9862-9867.
18. Lukomski, S., Hoe, N. P., Abdi, I., Rurangirwa, J., Kordari, P., Liu, M., Dou, S. J., Adams, G. G., and Musser, J. M. (2000) Infect Immun 68, 535-42.

## Claims

1. An assay method for an anti-bacterial agent comprising:
(a) providing as a first component protein SIC (streptococcal inhibitor of complement);
(b) providing as a second component an antibacterial peptide selected from α-defensin, LL-37 or a mixture thereof;
(c) contacting the first component with a test substance in the presence of the second component; and
(d) determining the interaction or activity of the first component with the second component to determine thereby whether a test substance is an effective anti-bacterial agent.

2. A method according to claim 1 wherein the first component protein SIC comprises wild type protein SIC derived from *S. pyogenes,* a fragment thereof which maintains the ability to inactivate human neutrophil α-defensin and/or LL-37, or a functional variant of protein SIC having at least 70% sequence identity to protein SIC having the amino acid sequence of SEQ ID NO: 1 and which maintains the ability to inactivate human neutrophil α-defensin and/or LL-37.

3. A method according to claim 2 wherein protein SIC is derived from *S. pyogenes* of serotype selected from M1, M12, M57 or M55.

4. A method according to claim 2 wherein protein SIC is derived from *S. pyogenes* of M1 serotype.

5. A method according to claim 4 wherein protein SIC has the sequence of SEQ ID NO: 1.

6. A method according to any one of claims 1 to 5 wherein step (d) comprises monitoring the interaction of the antibacterial peptide with protein SIC.

7. A method according to any one of claims 1 to 5 wherein step (d) comprises monitoring the activity of the antibacterial peptide.

8. A method according to claim 7 wherein step (d) comprises contacting the assay sample with bacteria to determine whether the antibacterial peptide has retained antibacterial activity.

9. A method according to claim 8 wherein step (a) comprises providing bacteria which express protein SIC and wherein step (d) comprises monitoring the growth of the bacteria in the presence of a test substance.

10. A method according to claim 8 or 9 wherein the method comprises determining the amount of an additional antibacterial peptide which is required to confer antibacterial activity in the presence of the test substance, to thereby determine the residual activity of the antibacterial peptide provided in step (b).

11. A method according to any one of the preceding claims further comprising the step of formulating an agent identified as an inhibitor of protein SIC activity as a pharmaceutical composition with a pharmaceutically acceptable carrier.

## Patentansprüche

1. Testverfahren für ein antibakterielles Mittel, umfassend:
(a) das Bereitstellen von Protein SIC (Streptokokken-Inhibitor vom Komplement) als erste Komponente;
(b) das Bereitstellen eines antibakteriellen Peptids, das aus α-Defensin, LL-37 oder einem Gemisch davon ausgewählt ist, als zweite Komponente;
(c) das Kontaktieren der ersten Komponente mit einer Testsubstanz in Gegenwart der zweiten Komponente; und
(d) das Bestimmen der Wechselwirkung oder der Aktivität der ersten Komponente mit der zweiten Komponente, um **dadurch** festzustellen, ob eine Testsubstanz ein wirksames antibakterielles Mittel darstellt.

2. Verfahren nach Anspruch 1, wobei das als erste Komponente dienende Protein SIC folgendes umfasst: Wildtypprotein SIC, abgeleitet von S. pyogenes, ein Fragment davon, bei dem die Fähigkeit zur Inaktivierung von humanem, neutrophilem α-Defensin und/oder LL-37 aufrecht erhalten bleibt, oder eine funktionelle Variante von Protein SIC die eine mindestens 70 %-ige Sequenz-Identität zu dem die Aminosäuresequenz SEQ ID NO: 1 aufweisenden Protein SIC besitzt und bei der die Fähigkeit zur Inaktivierung von humanem neutrophilem α-Defensin und/oder LL-37 aufrecht erhalten bleibt.

3. Verfahren nach Anspruch 2, wobei das Protein SIC vom Serotyp von S. pyogenes, der aus M1, M12, M57 oder M55 ausgewählt ist, abgeleitet ist.

4. verfahren nach Anspruch 2, wobei das Protein SIC vom Serotyp M1 von S. pyogenes abgeleitet ist.

5. Verfahren nach Anspruch 4, wobei das Protein SIC die Sequenz von SEQ ID NO:1 aufweist,

6. Verfahren nach einem der Ansprüche 1 bis 5, wobei die Stufe (d) das Überwachen der Wechselwirkung des antibakteriellen Peptids mit Protein SIC umfasst.

7. Verfahren nach einem der Ansprüche 1 bis 5, wobei die Stufe (d) die Überwachung der Aktivität des antibakteriellen Peptids umfasst.

8. Verfahren nach Anspruch 7, wobei die Stufe (d) das Kontaktieren der Testprobe mit Bakterien umfasst, um festzustellen, ob das antibakterielle Peptid seine antibakterielle Aktivität behalten hat.

9. Verfahren nach Anspruch 8, wobei die Stufe (a) das Bereitstellen von Bakterien umfasst, die Protein SIC exprimieren, und wobei die Stufe (d) das Überwachen des Wachstums der Bakterien in Gegenwart einer Testsubstanz umfasst.

10. Verfahren nach Anspruch 8 oder 9, wobei das Verfahren das Bestimmen der Menge eines zusätzlichen antibakteriellen Peptids umfasst, das zum Verleihen von antibakterieller Aktivität in Gegenwart der Testsubstanz erforderlich ist, um **dadurch** die restliche Aktivität des in Stufe (b) bereitgestellten antibakteriellen Peptids zu bestimmen.

11. Verfahren nach einem der vorstehenden Ansprüche, ferner umfassend die Stufe der Zubereitung eines Mittels, das als ein Inhibitor von Protein SIC-Aktivität identifiziert worden ist, in Form einer pharmazeutischen Zusammensetzung zusammen mit einem pharmazeutisch verträglichen Träger.

## Revendications

1. Procédé de dosage pour un agent antibactérien comprenant :
(a) l'apport, en tant que premier composant, d'une protéine SIC (inhibiteur streptococcique du complément) ;
(b) l'apport, en tant que second composant, d'un peptide antibactérien choisi parmi la α-défensine, le LL-37 ou un mélange de ceux-ci ;
(c) le contact du premier composant avec une substance de test en présence du second composant ; et
(d) la détermination de l'interaction ou de l'activité du premier composant avec le second composant pour déterminer de cette façon si une substance de test est un agent antibactérien efficace.

2. Procédé selon la revendication 1, dans lequel le premier composant protéine SIC comprend une protéine SIC du type sauvage dérivée de *S. pyogenes,* un fragment de celle-ci qui conserve la capacité à inactiver la α-défensine des neutrophiles humains et/ou le LL-37, ou un variant fonctionnel de la protéine SIC ayant une identité de séquence d'au moins 70 % par rapport à la protéine SIC ayant la séquence d'acides aminés de SEQ ID NO: 1 et qui conserve la capacité à inactiver la α-défensine des neutrophiles humains et/ou le LL-37.

3. Procédé selon la revendication 2, dans lequel la protéine SIC est dérivée de *S. pyogenes* d'un sérotype choisi parmi M1, M12, M57 ou M55.

4. Procédé selon la revendication 2, dans lequel la protéine SIC est dérivée de *S. pyogenes* de sérotype M1.

5. Procédé selon la revendication 4, dans lequel la protéine SIC a la séquence de SEQ ID NO: 1.

6. Procédé selon l'une quelconque des revendications 1 à 5, dans lequel l'étape (d) comprend le contrôle de l'interaction du peptide antibactérien avec la protéine SIC.

7. Procédé selon l'une quelconque des revendications 1 à 5, dans lequel l'étape (d) comprend le contrôle de l'activité du peptide antibactérien.

8. Procédé selon la revendication 7, dans lequel l'étape (d) comprend le contact de l'échantillon de dosage avec des bactéries pour déterminer si le peptide antibactérien a conservé une activité antibactérienne.

9. Procédé selon la revendication 8, dans lequel l'étape (a) comprend l'apport de bactéries qui expriment la protéine SIC et dans lequel l'étape (d) comprend le contrôle de la croissance des bactéries en présence d'une substance de test.

10. Procédé selon la revendication 8 ou 9, dans lequel le procédé comprend la détermination de la quantité d'un peptide antibactérien supplémentaire qui est nécessaire pour conférer une activité antibactérienne en présence de la substance de test pour déterminer de cette façon l'activité résiduelle du peptide antibactérien fourni à l'étape (b).

11. Procédé selon l'une quelconque des revendications précédentes, comprenant en outre l'étape consistant à formuler un agent identifié comme étant un inhibiteur de l'activité de la protéine SIC en tant que composition pharmaceutique avec un véhicule pharmaceutiquement acceptable.


### SEQUENCE LISTING

<110> HANSA MEDICAL RESEARCH AB
<120> ASSAY
<130> N87287A SER/SJB
<150> GB 0303809.8
   <151> 2003-02-19
<160> 5
<170> PatentIn version 3.1
<210> 1
   <211> 305
   <212> PRT
   <213> S. pyogenes
<400> 1
   Met Asn Ile Arg Asn Lys Ile Glu Asn Ser Lys Thr Leu Leu Phe Thr
   1               5                   10                  15
   Ser Leu Val Ala Val Ala Leu Leu Gly Ala Thr Gln Pro Val Ser Ala
               20                  25                  30
   Glu Thr Tyr Thr Ser Arg Asn Phe Asp Trp Ser Gly Asp Asp Trp Pro
           35                  40                  45
   Glu Asp Asp Trp Ser Gly Asp Gly Leu Ser Lys Tyr Asp Arg Ser Gly
       50                  55                  60
   Val Gly Leu Ser Gln Tyr Gly Trp Ser Gln Tyr Gly Trp Ser Ser Asp
   65                  70                  75                  80
   Lys Glu Glu Trp Pro Glu Asp Trp Pro Glu Asp Asp Trp Ser Ser Asp
                   85                  90                  95
   Lys Lys Asp Glu Thr Glu Asp Lys Thr Arg Pro Pro Tyr Gly Glu Ala
               100                 105                 110
   Leu Gly Thr Gly Tyr Glu Lys Arg Asp Asp Trp Gly Gly Pro Gly Thr
           115                 120                 125
   Val Ala Thr Asp Pro Tyr Thr Pro Pro Tyr Gly Gly Ala Leu Gly Thr
   130 135 140
   Gly Tyr Glu Lys Arg Asp Asp Trp Gly Gly Pro Gly Thr Val Ala Ile
   145 150 155 160
   Asp Pro Tyr Thr Pro Pro Tyr Gly Glu Ala Leu Gly Thr Gly Tyr Glu
   165 170 175
   Lys Arg Asp Asp Trp Arg Gly Pro Gly His Ile Pro Lys Pro Glu Asn
   180 185 190
   Glu Gln Ser Pro Asn Pro Ser His Ile Pro Glu Pro Pro Gln Ile Glu
   195 200 205
   Trp Pro Gln Trp Asn Gly Phe Asp Glu Leu Ser Phe Gly Pro Ser Asp
   210 215 220
   Trp Gly Gln Ser Glu Asp Ala Pro Arg Phe Pro Ser Glu Pro Arg Val
   225 230 235 240
   Pro Glu Lys Pro Gln His Thr Pro Gln Lys Asn Pro Gln Glu Ser Asp
   245 250 255
   Phe Asp Arg Gly Phe Ser Ala Gly Leu Lys Ala Lys Asn Ser Gly Arg
   260 265 270
   Gly Ile Asp Phe Glu Gly Phe Gln Tyr Gly Gly Trp Ser Asp Glu Tyr
   275 280 285
   Lys Lys Gly Tyr Met Gln Ala Phe Gly Thr Pro Tyr Thr Pro Ser Ala
   290 295 300
   Thr
   305
<210> 2
   <211> 915
   <212> DNA
   <213> S. pyogenes
<400> 2
   atgaatatta gaaataagat tgaaaatagt aaaacactac tatttacatc ccttgtagcc     60
   gtggctctac taggagctac acaaccagtt tcagccgaaa cgtatacatc acgcaatttt    120
   gactggtctg gagatgactg gcctgaagat gactggtctg gagatggttt gtctaaatat    180
   gaccggtctg gagttggttt gtctcaatat ggctggtctc aatatggctg gtctagcgat    240
   aaagaagaat ggcctgaaga ttggcctgaa gatgactggt ctagcgataa aaaagatgag    300
   acagaagata aaacgagacc accatatgga gaagcattag gtacagggta tgaaaaacgt    360
   gatgattggg gaggacctgg tacggtggca actgaccctt acactccacc atatggagga    420
   gcattaggta cagggtatga aaaacgtgat gattggggag gacctggtac ggtagcaatt    480
   gacccttaca ctccaccata tggagaagca ttaggtacag ggtatgaaaa acgtgatgat    540
   tggagaggac ctggacatat tcctaaacct gagaacgaac aatcaccaaa cccatctcat    600
   attcctgaac ctcctcagat tgagtggcct cagtggaatg gctttgatga attatcattt    660
   ggcccctctg attggggcca atctgaggac gcccctcgtt tcccaagtga acctcgtgtg    720
   ccagaaaaac cgcaacatac tcctcaaaaa aatccacaag aatcagattt tgatagaggg    780
   ttttcagctg gcttgaaagc aaaaaactca ggtagaggta ttgattttga aggtttccag    840
   tatggtggct ggtcagacga atataaaaaa ggttacatgc aagccttcgg tacaccatat    900
   acaccatcag caacg                                                     915
<210> 3
   <211> 30
   <212> PRT
   <213> Homo sapiens
<400> 3
   Ala Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 4
   <211> 37
   <212> PRT
   <213> Homo sapiens
<400> 4
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35
<210> 5
   <211> 28
   <212> PRT
   <213> Homo Sapiens
<400> 5
   Gly Cys Pro Arg Asp Ile Pro Thr Asn Ser Pro Glu Leu Glu Glu Thr
   1 5 10 15
   Leu Thr His Thr Ile Thr Lys Leu Asn Ala Glu Asn
   20 25